# EUROPEAN PATENT APPLICATION

(11) **EP 2 025 284 A1**
(43) Date of publication of application: **18.02.2009**
(21) Application number: 07743285.4
(22) Date of filing: 14.05.2007
(51) Int. Cl.: A61B 5/00, G06F 3/048

(54) **DISPLAY PROCESSING DEVICE**

(30) Priority: 05.06.2006 JP 2006155924
(71) Applicant: Konica Minolta Medical & Graphic, Inc., Hino-shi, Tokyo 191-8511 (JP)
(72) Inventor: KATSUSHIMA, Kazuhiko, Hachioji-shi, Tokyo 192-8505 (JP)
(74) Representative: Henkel, Feiler & Hänzel
(86) International application number: PCT/JP2007/059850
(87) International publication number: WO 2007/141995

(57) **Abstract**

A display processing device where operation for displaying medical images is facilitated and smooth. In the display processing device, display layout information determined corresponding to region segments set for a display region is stored in a storage section. When an operation section specifies at which position on the display screen a medical image is to be displayed, a control section determines a region segment in the display region to which the specified display position belongs, and reads, from the storage section, display layout information corresponding to the region segment. Then, according to the read display layout information, the control section splits the display region and displays the medical image, and changes the display layout of the medical image.

## Description

### Technical Field

The present invention relates to a display processing device for display processing of a medical image.

### Background Art

As for a medical image obtained when a patient is photographed for an examination, usually a plurality of medical images are photographed in one examination, changing photographic method and photographic device. When photography by Computed Tomography (CT) or Magnetic Resonance Imaging (MRI) is performed, nearly 100 medical images are obtained in one shooting.

When a plurality of such medical images are displayed on a monitor and interpreted, usually the images are compared and interpreted with a plurality of medical images simultaneously displayed on one screen by, for example, splitting a display region of a display screen of a monitor into a plurality of display regions and placing a medical image in each region.

As a technique of a display layout which is easy to examine, a method is disclosed where a plurality of images displayed in a display region with a random size and position is allocated in a preregistered layout pattern and redisplayed (for example, see Patent Document 1). There is also a technique where an image can be moved to a desired position between different display regions to be displayed (for example, see Patent Document 2).
Patent Document 1: Japanese Patent Application Laid-Open Publication No. H5-74750
Patent Document 2: Japanese Patent Application Laid-Open Publication No. H5-66912

### Disclosure of the Invention

### Problems to be Solved by the Invention

However, with the method described in the above-mentioned Patent Document 1, the displayed medical images are automatically allocated in the layout pattern, therefore, a doctor cannot select the position to display the medical image when the images are displayed on a split display. Consequently, a desired layout cannot be realized.
With the method described in the above-mentioned Patent Document 2, since the medical images can be placed in a desired position beyond the set display region, degree of freedom is high, however, position and display size of each medical image needs to be specified by operation one by one. When there is a large number of medical images to be interpreted, such operation is troublesome and ease of operation rather decreases.

An object of the present invention is to provide a display processing device which realizes easy and smooth display operation when a plurality of medical images are displayed.

### Means for Solving the Problem

According to the invention described in claim 1, there is provided a display processing device comprising:
a display section to provide one display region or a plurality of display regions on a display screen to display a medical image in each of the display regions;
a segment setting section to divide the display region to a plurality of regions and to set a region segment;
a storage section to store display layout information of the medical image specified corresponding to the region segment;
an operation section to specify a display position of the medical image to be displayed on the display screen; and
a display control section to determine the region segment of the display region to which the display position specified by the operation section belongs and to change a display layout of the medical image on the display screen according to the display layout information corresponding to the region segment.

### Advantageous Effect of the Invention

According to the invention described in claim 1, the display layout can be automatically changed according to the display operation of the medical image to be displayed. Therefore, when a plurality of medical images are displayed, a user does not have to perform special operation for a desired display layout, and display operation can be performed easily and smoothly.

According to the invention in claim 2 and 3, by specifying the splitting method or the split region where the medical image is placed to visually link the display operation of the medical image with the changed result of the layout in the display layout information, the user can determine, for example, the display position of the medical image while predicting the display layout changed by the display operation and operation can be performed easily.

According to the invention in claim 4, not only display of a plurality of medical images by splitting the display region can be performed but the medical image to be displayed can be replaced with the medical image already displayed and then displayed, therefore, various display forms corresponding to layout change can be performed.

### Brief Description of the Drawings

FIG. 1 is a diagram showing a structure of a display processing device of the preferred embodiments;
FIG. 2 is a diagram showing an example of display layout information;
FIG. 3 is a diagram showing an example of a display screen;
FIG. 4 is a diagram showing a region segment set in a display region of a medical image;
FIG. 5 is a flowchart describing display processing of the medical image performed by the display processing device;
FIG. 6 is a diagram showing split regions after image display section d32 of the display screen is split in two vertically;
FIG. 7 is a diagram showing an example of splitting in two vertically and the region segment set in each split region after splitting;
FIG. 8 is a diagram showing an example of after splitting in two vertically, one of the split regions is further split in two horizontally, and the region segment set in each split region after splitting;
FIG. 9 is a diagram showing an example of an icon of a pointer; and
FIG. 10 is a diagram showing an example of a display screen when a split region is combined.

### Description of Reference Numerals

- 10: display processing device
- 11: control section
- 12: operation section
- 13: display section
- 14: storage section
- 15: communication section

### Best Mode for Carrying Out the Invention

First, a structure will be described.

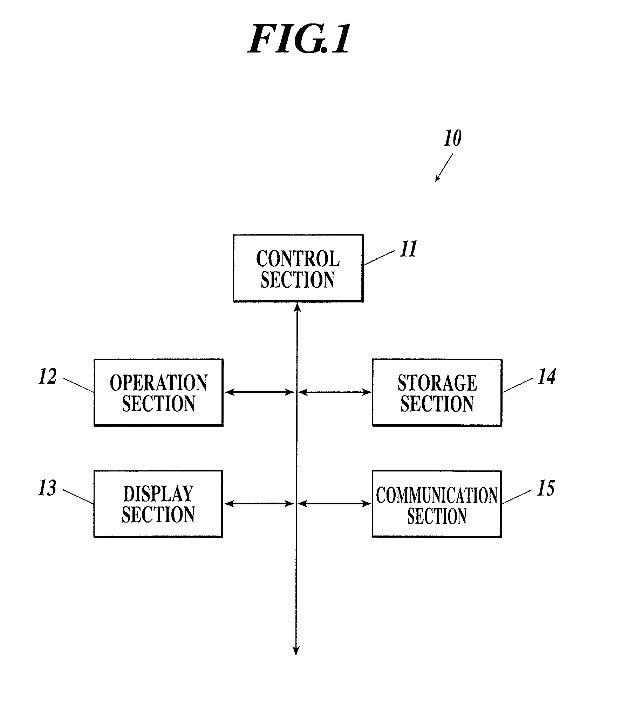

FIG. 1 shows a structure of a display processing device 10 of the present embodiment.
The display processing device 10 is a computer device for display processing of a medical image when the medical image is displayed, and as shown in FIG. 1, includes a control section 11, operation section 12, display section 13, storage section 14 and communication section 15.

The control section 11 includes a Central Processing Unit (CPU), Random Access Memory (RAM), etc. The control section 11 reads out various control programs stored in the storage section 14 and performs various computations according to the programs or centrally controls the sections 11 to 15.

The operation section 12 includes a keyboard, mouse, etc., and generates an operation signal according to operation of them to output to the control section 11.

The display section 13 includes a display and displays on the display various pieces of display information such as an operation screen or medical image according to an instruction by the control section 11.

The storage section 14 stores various control programs as well as parameter and data necessary for performing the programs, result of processing by the control section 11 and the like.
For example, the storage section 14 includes a layout table 141 necessary for performing a display processing program of the medical image.

The layout table 141 stores display layout information for displaying the medical image on the display screen.

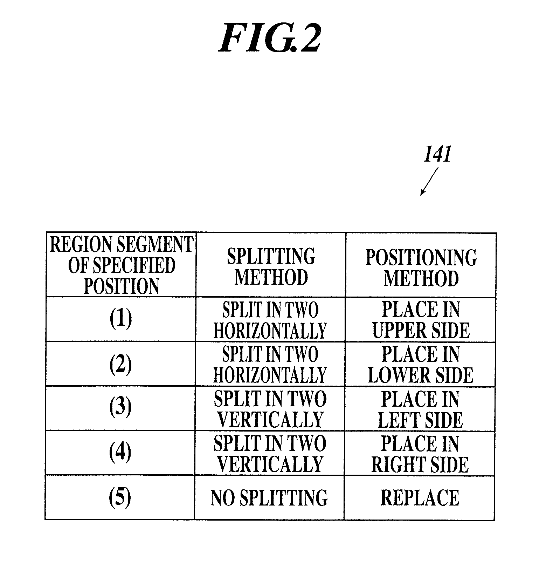

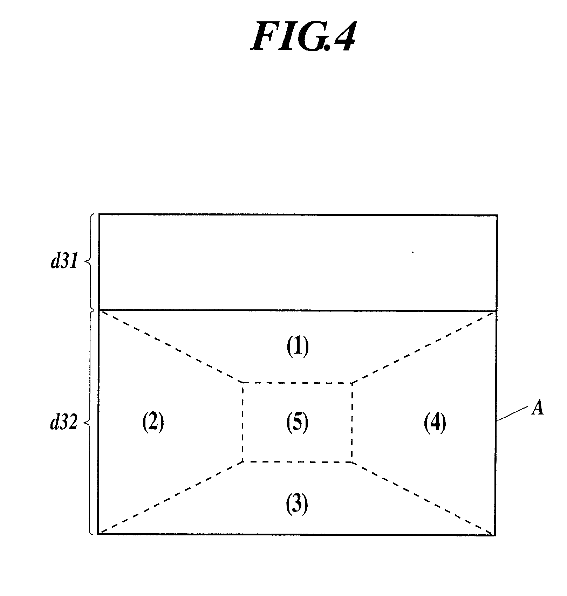

FIG. 2 shows an example of the layout table 141. In the example shown in FIG. 2, one display region is split in five regions (1) to (5) as shown in FIG. 4, and FIG. 2 shows an example of display layout information where splitting method of the display region and displaying method of medical image in the display region is predetermined according to which region segment among the five regions the display position of the medical image belongs to.
For example, in the layout table 141 of FIG. 2, when the display position of the medical image belongs to region segment "(1)", the specified displaying method is that the display region is split by the splitting method of "split in two horizontally" and between the split regions "place in upper side", in other words, the medical image to be displayed is placed in the upper split region.

Here, the display screen is an entire region of a display member such as a display, etc. where display can be performed. On the other hand, display region is a unit of display of the medical image displayed on the display screen such as a window where the medical image is displayed. As for the display region, one display region may spread on the entire display screen or the display screen may be split into a few regions to form a plurality of display regions.

In the display processing of the medical image, the display layout of the medical image is changed according to the display layout information stored in the layout table 141. Therefore, the display layout information is specified so that the layout is visually changed linked with the operation by the user. For example, as in the example shown in FIG. 2, when the region segment (1) in the upper portion of the display region is specified as the display position, as for a layout such as the layout of splitting the display region in two horizontally and placing the medical image to be displayed in the upper split region, the operation by the user (specifying the upper portion of the display region) and its display result which is the layout (medical image placed in the upper split region) visually matches and consequently the operation can be easier for the user.

The communication section 15 includes a communication interface such as a network interface card, etc., and performs communication with external devices on the network. For example, the communication section 15 receives a medical image to be displayed from the image server which stores the medical image.

Next, the operation of the display processing device 10 will be described.
When a doctor performs display instruction operation of a medical image through the operation section 12 to perform interpretation of the medical image, in the display processing device 10, the control section 11 controls the display section 13 to display a patient list on the display section 13. The patient list is a list of photographed medical images according to patient. When a doctor selects any one of the medical images from the patient list, the selected medical image is displayed on the display section 13 by the control of the control section 11.

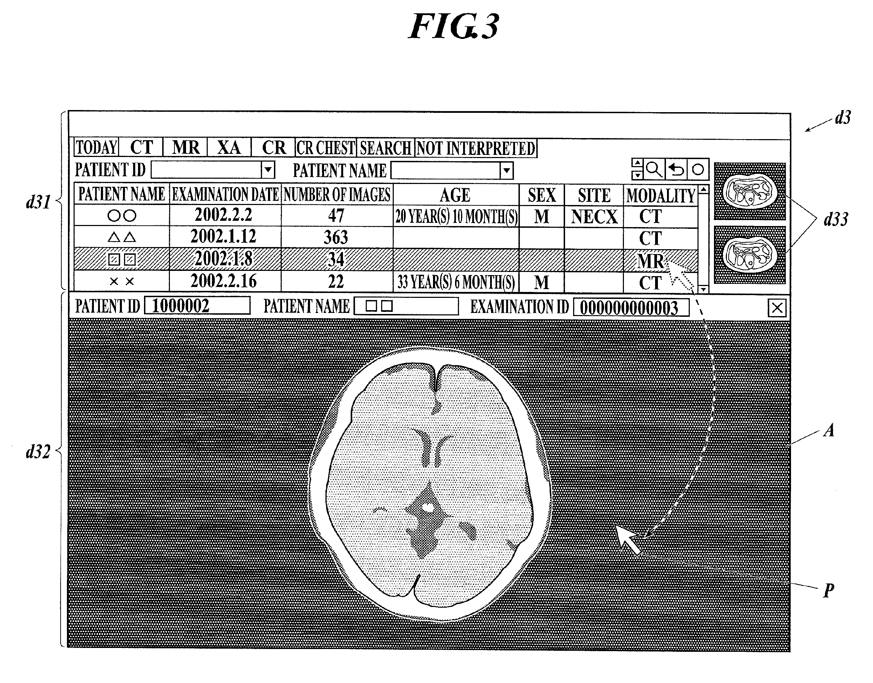

FIG. 3 shows an example of a display screen.
The display screen d3 shown in FIG. 3 includes a list display section d31 and an image display section d32. The patient list is displayed in the list display section d31 and a window A of the medical image selected from the patient list is displayed in the image display section d32. The window is a display region which is a unit of display of the medical image and one medical image is displayed in one window.

A related image of the medical image displayed in the list in the list display section d31 can be displayed in the thumbnail image d33. The related image is another medical image photographed in relation to the medical image such as another medical image photographed in a same examination with a different photographic condition.
One or a plurality of windows of medical images can be displayed simultaneously in the image display section d32. By the computation of the control section 11, an inner region of the window is previously split in five regions (1) to (5) as shown in FIG. 4 and the region segments are set. As for the region segments, area ratio of each of the region segments to the display region is previously set and the control section 11 sets the region segment according to the ratio. As described above, the display layout information is specified corresponding to the region segments (1) to (5) and stored in the layout table 141 of the storage section 14.

When a plurality of medical images are displayed in the display processing device 10, the control section 11 controls the display to divide the display region of the image display section d32 and place the window of the medical image in the split region in a way that the medical images are not displayed overlapped in order to enable interpretation by comparison. At this time, the control section 11 calculates the size of the window to match the region size of the split region to generate the window in that size and displays the window. The control section 11 constantly manages status such as display status of each of the windows or the split status in the display region in the entire image display section d32 by, for example, setting a coordinate in the display region of the image display section d32 and storing in the RAM of the control section 11 the coordinate values of the display position of the window displayed in the image display section d32.

When a plurality of medical images are to be displayed simultaneously from the status of display screen d3 shown in FIG. 3, the doctor performs the operation of specifying the medical image (hereinafter referred to as target image) to be displayed and specifying the position in which the target image is to be displayed. This specification operation can be done by pointer p displayed on the display screen d3. The target image is specified by dragging of the target image using the pointer p and the display position is specified by moving the pointer p to a desired position and dropping. The target image can be specified from the patient list or the group of thumbnail image d33 of the related image.

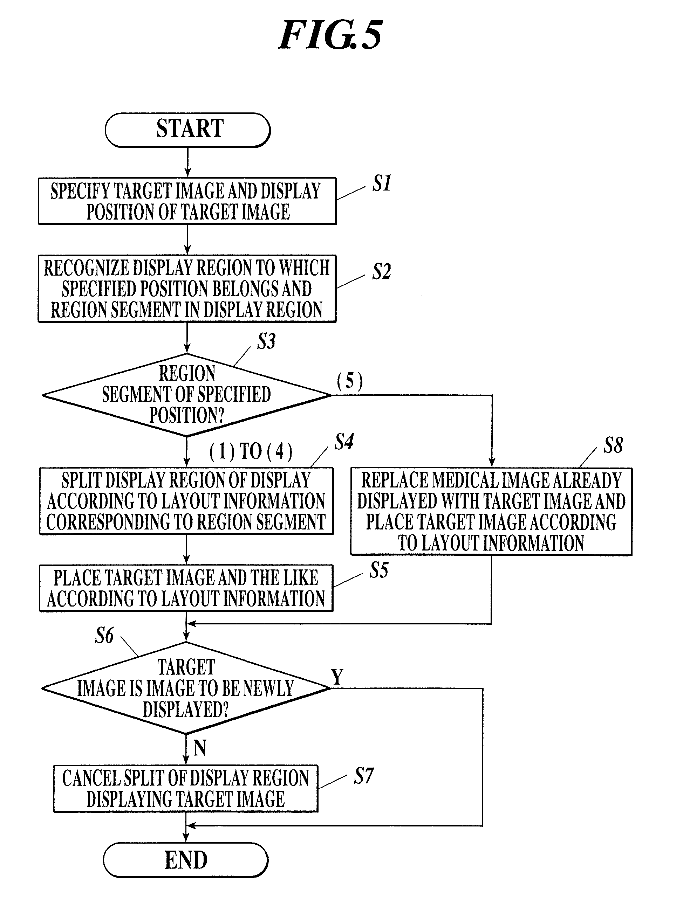

In the display processing device 10, the display processing shown in FIG. 5 starts according to the drag and drop of the target image.
In the display processing shown in FIG. 5, drag and drop operations of the target image is performed through the operation section 12, and when the specification operation of the target image and its display position is performed (step S1), from the coordinate value of the display position (hereinafter referred to as specified position) specified by the dropping, the control section 11 recognizes the window to which the specified position belongs and the region segment of the window to which the specified position belongs (step S2). In the example of the display screen shown in FIG. 4, the window to which the specified position belongs is recognized as window A, and the region segment (1) to (5) split in the window A is recognized.

Next, in order to perform the display processing according to the recognized region segment, the control section 11 determines which region segment was recognized (step S3). When the recognized region segment of the specified position is (1) to (4) (step S3; (1) to (4)), the processing advances to step S4, and when the region segment of the specified position is (5), (step S3; (5)), the processing advances to step S8.

First, a case where the region segment is (1) to (4) is described.
In this case, the control section 11 reads out the display layout information corresponding to the region segment (1) to (4) from the layout table 141. Then, the display region in the recognized window is split according to the splitting method specified in the read out display layout information (step S4). Here, a case where the region segment is (4) is described. When the region segment is (4), the splitting method of "split in two vertically" is specified in the layout table 141 shown in FIG. 2, and thus the control section 11 splits the display region of the window A in two vertically and reduces the window A according to the size of the split region. The window A after reduction is window A1. At the same time, a new window A2 is generated according to the size of the split region and the target image is displayed on the window A2.

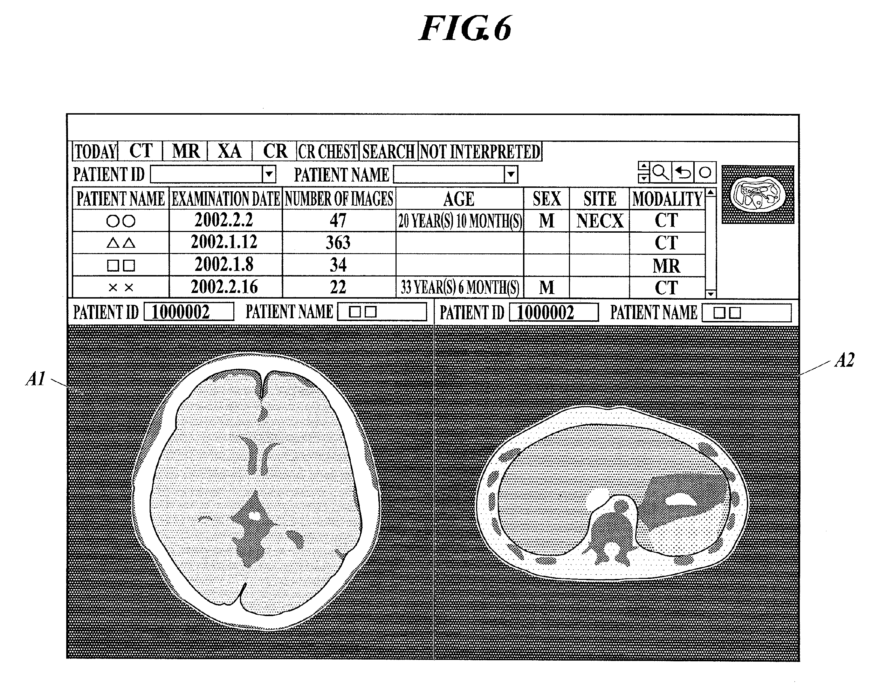

Next, the control section 11 places the window A2 of the target image in one of the split regions and window A1 in the other split region according to the displaying method specified by the display layout information (step S5). When the region segment is (4), the displaying method of the target image is specified to be "place in right side" in the layout table 141 shown in FIG. 2. Therefore, as shown in FIG. 6, between the split regions split in two vertically, the control section 11 places the window A2 of the target image in the right split region and the window A1 in the left split region.

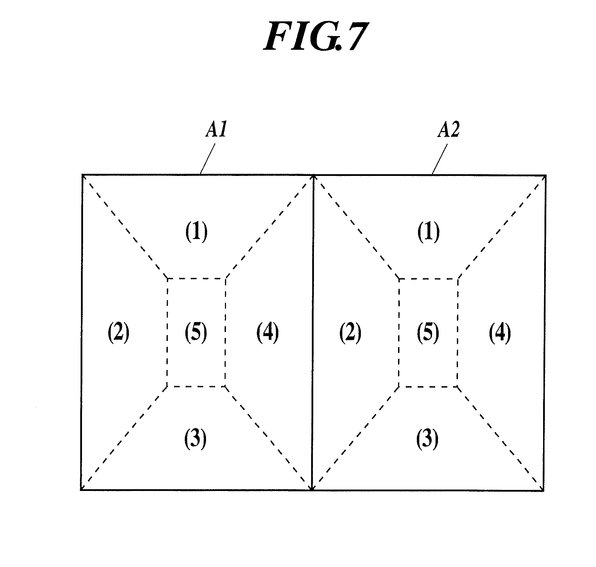

By repeating the processing of the above-described steps S1 to S5, a plurality of medical images can be displayed on the display screen. For example, as shown in FIG. 7, as for the windows A1 and A2 shown in FIG. 6, five region segments are set similar to window A. The region segments can be obtained by calculation by the control section 11 according to the size of the window. Therefore, display position of the target image can be newly specified in the display region of the windows A1 and A2.

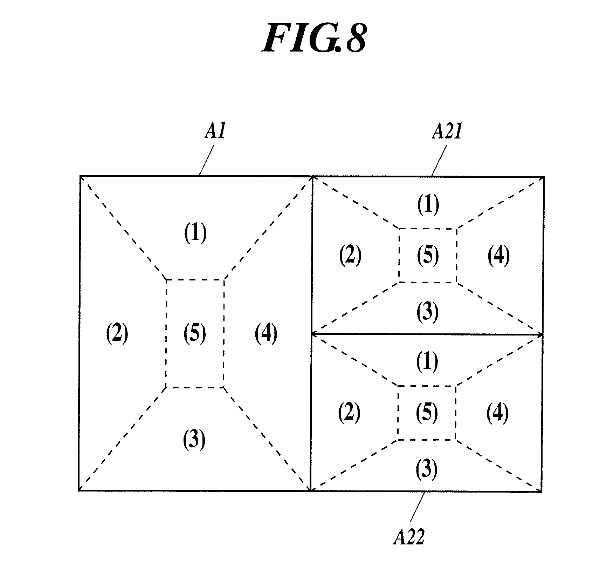

For example, when the display position is specified in the region segment (3) of the window A2, the control section 11 splits the display region of the window A2 in two horizontally as shown in FIG. 8 according to the display layout information, and displays window A21 which is the window A2 reduced according to the size of the split region and window A22 newly generated in order to display the target image. Then, the window A22 of the target image is placed in the lower split region specified by the display layout information and the window A21 is placed in the other split region.

Alternatively, instead of specifying a new target image as described above, a medical image on the window already displayed can also be specified as the target image. For example, when the medical image displayed in window A22 shown in FIG. 8 is dragged and dropped in the region segment (3) of the window A1, the display region of the window A1 is split in two horizontally according to the display layout information and the dragged medical image (target image) of window A22 is displayed in the lower split region.

After the target image is displayed, when the target image is a medical image to be newly displayed selected from the patient list or the thumbnail image (step S6; Y), the control section 11 does not perform any processing and the processing ends.
On the other hand, when the target image is a medical image already displayed on the window (step S6; N), the control section 11 cancels the split status of the window in which the target image was originally displayed (step S7). When the medical image of the window A22 is dropped in the window A1 as in the example described above, the split status of the windows A21 and A22 is cancelled. In other words, the window A22 is closed and the window A21 is enlarged to be displayed in the entire display region of windows A21 and A22.

Next, a case where the region segment of the specified position is (5) in step S3 is described.
In this case, the splitting method specified in the layout table 141 shown in FIG. 2 is "no splitting" and the displaying method is "replace". Therefore, the control section 11 replaces the medical image already displayed on the window to which the specified position belongs with the target image according to the display layout information and displays the target image (step S8).

For example, in the layout shown in FIG. 8, when the displayed image of the window A22 is dragged and dropped in the region segment (5) of the window A1, the medical image displayed in the window A1 is replaced with the target image displayed in the window A22 and displayed.

After the replacement, the processing advances to step S6. In other words, when the target image replaced and displayed is a medical image already displayed in any other window, the split status of the window in which the medical image was displayed is cancelled. In the above-described example, the window A22 is closed and the window A21 is enlarged to the entire display region of windows A21 and A22. As a result, the layout of the medical image is changed from the layout shown in FIG. 8 to the layout shown in FIG. 7.

As described above, according to the present embodiment, when a plurality of medical images are displayed, splitting and displaying according to the position specified for display of the target image is performed based on the display layout information. With this, along with the specification operation of the target image and its display position, display layout can be changed automatically to the display layout according to the specification operation. Consequently, when a plurality of medical images are displayed, a user does not have to perform special operation in order to obtain a desired layout, and easy and smooth display operation is possible.

Also, as for the display layout information, since the splitting method of the display region and the displaying method of the medical image are specified to link with the operation of the specification of the display position of the medical image, the user can perform the specification of the display position while visually predicting the layout after the specification and operability is enhanced.

As display layout information, by specifying displaying method of replacement, not only display of a plurality of medical images by splitting the display region but also display by replacing the medical image already displayed can be performed, therefore, layout change corresponding to various display operation can be performed.

The above-described embodiment is a suitable example of the present invention and the present invention is not limited to this example.
For example, an example where the medical image is replaced and displayed when applied to the region segment (5) is described, however, instead of replacement, a stacked display is possible, where the window of the target image is displayed overlapping on the window originally displaying a medical image.

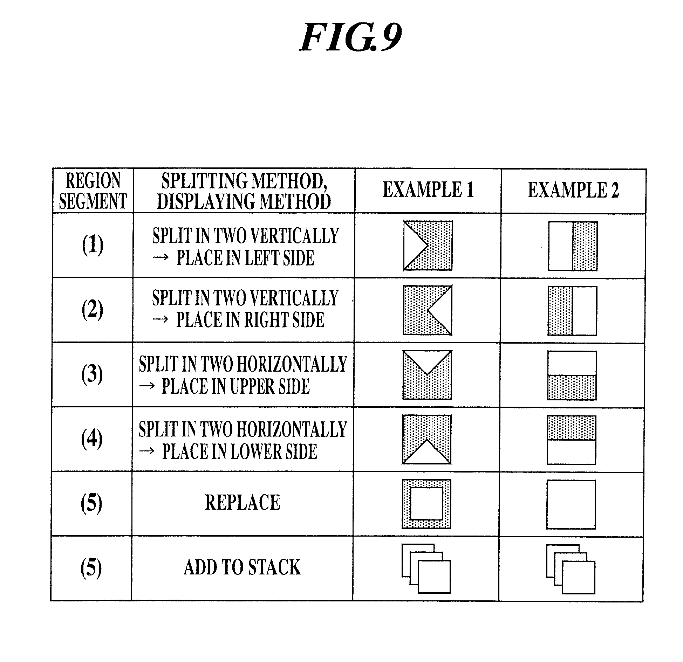

The icon shape of the pointer used when the target image is dragged and dropped can be shaped as shown in FIG. 9.
An example 1 shown in FIG. 9 is an example showing an icon shape formed so that the user can visually know in which region segment (1) to (5) the pointer is displayed. A rectangular icon is formed to show in which region segment (1) to (5) the pointer is positioned by adding color to a portion according to the shape of the region segment (1) to (5). As for region segment (5), two types of icon shapes are shown, a case of replacement and a case of stack display.

Alternatively, example 2 is an example showing an icon shape formed so that the user can visually know the changed result of the layout when the target image is dropped in the region segment (1) to (5) where the pointer is located. Here, a rectangular icon is formed to show the split status by setting the boundary of the split regions after splitting and to show in which split region the target image is placed by adding color to the split region.

These icons are displayed by display control by the control section 11. The control section 11 normally leaves the pointer in an arrow shape and when the pointer enters a window of a medical image, determines a position of the pointer in the display region to recognize the region segment to which the pointer position belongs. Then, the icon shape of the pointer is changed to the icon shape according to the region segment recognized and displayed.
As described above, with the icon shape according to the position of the pointer, the user can perform the dragging while predicting from the icon shape of the pointer the layout to be changed by the dropping, therefore, the operability enhances even more.

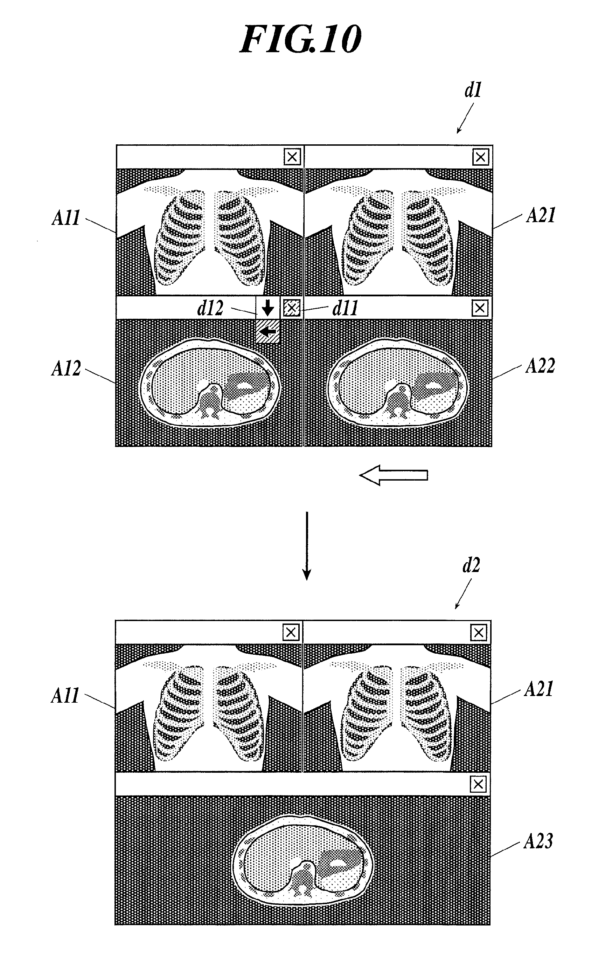

When a window in a split status is cleared, the split status can be cancelled, and a window in the other split region can be displayed enlarged to combine split regions. In this case, the combining direction can be specified when the window is cleared. For example, when operating an icon d11 for clearing an image provided in window A12 in a display screen d1 which is in a 2 x 2 split status as shown in FIG. 10, the control section 11 controls the display to display a pull-down menu d12. The pull-down menu d12 displays icons for selecting between the adjacent split regions (split region above or to the right) which split region is combined when the displayed image is cleared. The control section 11 judges the split region which can be combined from the display position of the window and displays the icon showing the combining direction.

In the pull-down menu d12, when the icon which shows combining with the split region on the right is operated, the control section 11 closes and clears the window A12 instructed to be cleared and cancels the split status with the split region to the right to display the window A22 enlarged on the entire display region of window A12 and A22. The result is display screen d2.
As described above, by judging the split region which can be combined when the displayed image is cleared and displaying the pull-down menu d12 for selecting the combining direction, the user can combine the split regions in the desired direction with easy operation, and smooth operation can be performed.

### Industrial Applicability

The present invention can be used in the technical field of displaying a medical image.

## Claims

1. A display processing device comprising:
a display section to provide one display region or a plurality of display regions on a display screen to display a medical image in each of the display regions;
a segment setting section to divide the display region to a plurality of regions and to set a region segment;
a storage section to store display layout information of the medical image specified corresponding to the region segment;
an operation section to specify a display position of the medical image to be displayed on the display screen; and
a display control section to determine the region segment of the display region to which the display position specified by the operation section belongs and to change a display layout of the medical image on the display screen according to the display layout information corresponding to the region segment.

2. The display processing device of claim 1, wherein:
in the display layout information, a splitting method of the display region is specified corresponding to the region segment; and
the display control section splits the display region to which the specified display position belongs according to the splitting method specified in the display layout information and displays the medical image to be displayed in any one of split regions.

3. The display processing device of claim 2, wherein:
in the display layout information, the split region in which the medical image is placed among the split regions split according to the splitting method is specified;
the display control section places, according to the display layout information, the medical image to be displayed in the specified split region among the split regions.

4. The display processing device of claim 1 or claim 2, wherein:
in the display layout information, a displaying method is specified corresponding to the region segment, the displaying method which replaces a medical image already displayed with the medical image to be displayed to display the medical image to be displayed; and
the display control section replaces the medical image already displayed in the display region to which the specified display position belongs with the medical image to be displayed to display the medical image to be displayed according to the display layout information.
